(19) Europäisches Patentamt / European Patent Office / Office européen des brevets

(11) **EP 3 919 908 A1**

(12) **EUROPEAN PATENT APPLICATION**
published in accordance with Art. 153(4) EPC

(43) Date of publication:
**08.12.2021 Bulletin 2021/49**

(21) Application number: **20747920.5**

(22) Date of filing: **28.01.2020**

(51) Int Cl.:
*G01N 33/543* (2006.01)     *G01N 33/544* (2006.01)

(86) International application number:
**PCT/JP2020/003029**

(87) International publication number:
**WO 2020/158750 (06.08.2020 Gazette 2020/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.01.2019 JP 2019015565**

(71) Applicant: **Asahi Kasei Kabushiki Kaisha Tokyo 1000006 (JP)**

(72) Inventors:
• **JOMORI, Daisuke**
  **Tokyo 100-0006 (JP)**
• **MATSUSE, Takeshi**
  **Tokyo 100-0006 (JP)**
• **HORII, Atsushi**
  **Tokyo 100-0006 (JP)**

(74) Representative: **dompatent von Kreisler Selting Werner - Partnerschaft von Patent- und Rechtsanwälten mbB Deichmannhaus am Dom Bahnhofsvorplatz 1 50667 Köln (DE)**

(54) **ABSORBENT PAD FOR IMMUNOCHROMATOGRAPHIC DIAGNOSIS KIT**

(57)     Provided is an absorbent pad for an immunochromatographic diagnosis kit to be used for an immunochromatographic diagnosis kit which has reduced variation in color intensity and excellent reproducibility. The present invention relates to the absorbent pad for an immunochromatographic diagnosis kit, the absorbent pad comprising nonwoven fabric that is composed of thermoplastic resin fiber and being characterized in that the nonwoven fabric has a weight of 20 to 200 g/m$^2$, a thickness of 0.06 to 1.20 mm, an average fiber diameter of 0.7 to 5.0 $\mu$m, an average pore diameter of 2.0 to 15.0 $\mu$m, and a degree of hydrophilization of 50 to 180 mm. The present invention also relates to an immunochromatographic diagnosis kit using the same.

FIG. 1

Liquid development direction

EP 3 919 908 A1

**Description**

FIELD

The present invention relates to an absorbent pad for an immunochromatographic diagnosis kit, and to an immunochromatographic diagnosis kit using it.

BACKGROUND

With changes in the situation of medicine in recent years, POCT (Point Of Care Testing) has become an increasing focus of interest. POCT is examination carried out for a subject by a health care professional or the subject themselves, providing the advantages of not only shortening examination time but also allowing test results to be directly confirmed by the subject. Because of these advantages, adoption of POCT has been steadily growing at hospital outpatient and ward facilities, as well as at clinics and in home medical care settings. Unlike testing at hospital central laboratories and outsourced examination centers, POCT tests are carried out at clinics and home treatment locations where the testers usually have insufficient knowledge regarding examination and are unfamiliar with measurement concepts. POCT testing reagents must therefore be simple to operate and give reliable test results when carried out by anyone with only minimal explanation.

Many widely used reagents for such simple and rapid diagnosis are test kits that utilize immunochromatographic measurement principles. The kits are constructed primarily with a chromatographic medium, a sample addition pad, a labeling reagent holder (conjugate pad) and an absorber (absorbent pad), the members being mounted on a base (backing sheet) and housed in a housing member, as necessary. Chromatographic media comprising nitrocellulose serve to develop liquid samples, while also serving as assessment units for test results. The labeling reagent holder (conjugate pad) containing the sample addition pad and the labeling reagent is situated at one end of the chromatographic medium in the lengthwise direction to allow flow of liquid, while the absorber (absorbent pad) is provided at the edge on the other side to absorb excess sample solution.

Examination by an immunochromatographic diagnosis kit is carried out by adding different harvested specimens or sample solutions to the sample addition pad. The added specimens or sample solutions pass through the labeling reagent holder, migrating through the chromatographic medium by capillary movement in the lengthwise direction and through the assessment unit, and being absorbed into the absorbent pad. When passing through the labeling reagent holder, substances to be detected in the sample solution form complexes with gold colloid or another colored labeling reagent by antigen-antibody reaction, and are then captured on the chromatographic medium in the assessment unit. For examination by an immunochromatographic diagnosis kit, the amount of substance to be detected that has been trapped in the assessment unit of the chromatographic medium during a specified reaction time (10 to 15 minutes) is visually determined using the intensity of coloration from the labeling reagent as the index.

Examination by immunochromatography has long been a highly desired goal for rapid and accurate disease assessment. Several test items are useful for diagnosis of acute coronary syndrome, for example. Acute myocardial infarction, as one type of acute coronary syndrome, is a disease in which the coronary arteries become obstructed by thrombus leading to necrosis of cardiac muscle tissue, and its prognosis is greatly affected by the success or failure of reperfusion at early onset. Highly accurate diagnosis is therefore important. In this case it is necessary to quantitatively detect markers necessary for diagnosis, and this means that the immunochromatographic diagnosis kit used for diagnosis naturally must have minimal variation in color intensity within the immunochromatographic diagnosis kit product. Using an immunochromatographic diagnosis kit with large variation in color intensity will naturally result in possible misdiagnosis, which in the case of the disease mentioned above can potentially be a matter of life or death. Minimizing variation in immunochromatographic diagnosis kit products is therefore a major issue considered in development of immunochromatographic diagnosis kits.

One cause of variation is thought to be the influence of the members composing the kit, and in particular the absorbent pad which serves to absorb liquid, because it is a cause of variation in color intensity or developing time due to variations in sample liquid absorption speeds, or variation in color intensity due to problems with background coloration.

The term "background" refers to the phenomenon of coloration of the nitrocellulose membrane when the labeling reagent clogs the pores of the nitrocellulose membrane or participates in nonspecific adsorption. A poor background reduces the contrast between the nitrocellulose membrane and the test lines (TL), making the colored TL lines less visible and interfering with assessment at medical facilities. A poor background also makes it difficult to judge negative TL (i.e. lack of coloration). These phenomena can potentially lead to false diagnosis.

Conventional absorption pads used in immunochromatographic test kits include cotton, nonwoven fabrics and filter paper composed of fibers such as cellulose fibers, glass fibers or pulp. Absorbent pads composed only of these materials, however, have inadequate water absorbing properties depending on their structure, constituent materials and production methods, or may also have variation in absorption amounts.

In order to solve these problems, PTL 1 has disclosed using an absorbent pad employing super-absorbent polymer particles, and PTL 2 has disclosed using an absorbent pad obtained by spraying silica particles on a nonwoven fabric material made of cellulose or other fibers. With these absorbent pads, however, it has been difficult to achieve uniform fiber density and uniform spraying amounts, and although they have improved water absorbing properties, different products tend to exhibit variation in moisture absorption speed, such that improvement in the variation between products has still not been realized.

Problems have also been reported in terms of the kit production steps, due to conventional absorbent pads. Long glass fiber absorbent pads, for example, form dust from shed fibers when they are cut during the production steps for immunochromatographic diagnosis kits. Because inhalation of such long glass fiber dust can potentially cause bronchial inflammation, these are not desirable for use from the viewpoint of health damage to production workers. Another reported problem is chipping of cutting module blades. For these reasons it is preferable to avoid the use of long glass fiber absorbent pads.

For a cellulose absorbent pad, on the other hand, when the immunochromatography kit is housed in its final product form, thickness variation may cause it to be thicker than designed and to fail to close, or to become thinner and be susceptible to problems such as positional shifting, both of which situations lead to variation between immunochromatographic diagnosis kit products.

With recent trends toward producing more compact and automated kits, there is growing demand for thinner and narrower absorbent pad-containing kits. Conventional cellulose absorbent pads, however, cannot be cut to thin sizes because of problems with their thickness and elastic modulus. With long glass fiber absorbent pads as well, their inconsistencies lead to variation in liquid absorption when they are thinly cut, also resulting in the problem of variation between immunochromatographic diagnosis kit products.

A high demand therefore exists for an absorbent pad that can be produced in compact form while limiting the maximum extent of variation.

[CITATION LIST]

[PATENT LITERATURE]

    [PTL 1] Japanese Patent Publication No. 6134615
    [PTL 2] Japanese Unexamined Patent Publication No. 2012-189346

SUMMARY

[TECHNICAL PROBLEM]

In light of the problems of the prior art described above, it is an object of the invention to provide an absorbent pad for an immunochromatographic diagnosis kit, for use in an immunochromatographic diagnosis kit having low variation in color intensity and excellent reproducibility.

[SOLUTION TO PROBLEM]

After much diligent experimentation with the goal of solving the aforementioned problem, the present inventors have found, unexpectedly, that by using an absorbent pad that is a nonwoven fabric composed of thermoplastic resin fibers having specific properties that has been subjected to hydrophilic treatment to render it hydrophilic, its liquid absorption performance becomes uniform and variation in the color intensity of an immunochromatographic diagnosis kit using it is vastly improved, and we have thereupon completed the present invention.

Specifically, the present invention is as follows.

    [1] An absorbent pad for an immunochromatographic diagnosis kit comprising a nonwoven fabric composed of thermoplastic resin fibers, wherein the nonwoven fabric has a basis weight of 20 to 200 g/m$^2$, a thickness of 0.06 mm to 1.20 mm, a mean fiber diameter of 0.7 $\mu$m to 5.0 $\mu$m, an average pore size of 2.0 $\mu$m to 15.0 $\mu$m and a degree of hydrophilization of 50 mm to 180 mm.

    [2] The absorbent pad for an immunochromatographic diagnosis kit according to [1] above, wherein the formation index of the nonwoven fabric is 90 or lower and the pore size distribution satisfies the following formulas:

$$Dmax/Dave < 2.00$$

$$Dmax/Dmin < 3.50$$

{where Dmax is the maximum pore size ($\mu$m), Dave is the average pore size ($\mu$m), and Dmin is the minimum pore size ($\mu$m)}.

[3] The absorbent pad for an immunochromatographic diagnosis kit according to [1] or [2] above, wherein the thermoplastic resin is any one or a combination of more than one from among polyethylene terephthalate, polybutylene terephthalate, polypropylene and polyethylene.

[4] The absorbent pad for an immunochromatographic diagnosis kit according to any one of [1] to [3] above, wherein the nonwoven fabric is a single-layer nonwoven fabric or a layered nonwoven fabric.

[5] An immunochromatographic diagnosis kit using an absorbent pad for an immunochromatographic diagnosis kit according to any one of [1] to [4] above.

[ADVANTAGEOUS EFFECTS OF INVENTION]

An immunochromatographic diagnosis kit using an absorbent pad for an immunochromatographic diagnosis kit of the invention has low variation in color intensity and excellent reproducibility.

An absorbent pad for an immunochromatographic diagnosis kit of the invention can exhibit low color intensity variation and excellent reproducibility if the nonwoven fabric composed of thermoplastic resin fibers is controlled to have a basis weight of 20 to 200 g/m², a thickness of 0.06 mm to 1.20 mm, a mean fiber diameter of 0.7 $\mu$m to 5.0 $\mu$m and an average pore size of 2.0 $\mu$m to 15.0 $\mu$m. An absorbent pad absorbs water, as its name suggests, and hydrophilic materials are used for its formation. Conventional cellulose absorbent pads or long glass fiber absorbent pads are hydrophilic materials, as are the absorbent pads described in PTLs 1 and 2. However, no effect of improvement in color intensity variation is seen with immunochromatographic diagnosis kits using these types of absorbent pads.

The present inventors have found, however, that in order to reduce variation in color intensity with an absorbent pad for an immunochromatographic diagnosis kit, it is important to use a hydrophobic thermoplastic nonwoven fabric, forming a uniform structure and also controlling the degree of hydrophilicity by hydrophilicizing treatment. Moreover, merely obtaining higher hydrophilicity is not enough to increase the moisture absorption speed. In other words, for improved moisture absorption speed it is necessary to control both the hydrophilicity and hydrophobicity, and using a hydrophobic thermoplastic nonwoven fabric in hydrophilicizing treatment according to the invention allows control of the water absorption power as specified by the invention and leads to an improved background. In addition, since this results in fewer shed fibers and easier cutting and shaping during the production steps for the immunochromatographic diagnosis kit, compared to long glass fibers, it increases the degree of size design freedom (including thickness and width) for the pad and kit, and can also ensure stability during the process.

BRIEF DESCRIPTION OF DRAWINGS

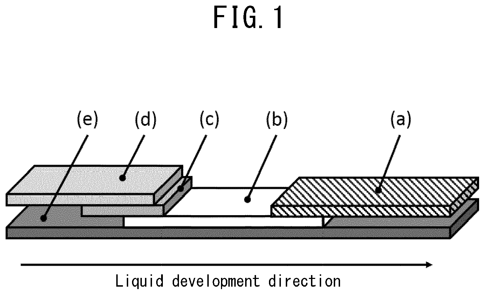

Fig. 1 is a cross-sectional view of an immunochromatographic diagnosis kit as one embodiment of the invention.

DESCRIPTION OF EMBODIMENTS

The invention will now be explained in detail using an embodiment thereof.

An absorbent pad for an immunochromatographic diagnosis kit according to the embodiment comprises a nonwoven fabric composed of thermoplastic resin fibers, wherein the nonwoven fabric has a basis weight of 20 to 200 g/m², a thickness of 0.06 mm to 1.20 mm, a mean fiber diameter of 0.7 $\mu$m to 5.0 $\mu$m, an average pore size of 2.0 $\mu$m to 15.0 $\mu$m and a degree of hydrophilization of 50 mm to 180 mm.

Examination methods based on immunochromatography carried out using immunochromatographic diagnosis kits generally include flow-through systems in which a solution containing a substance to be measured is passed through a membrane in the vertical direction, and lateral flow systems in which it is passed through in the horizontal direction, and the absorbent pad for an immunochromatographic diagnosis kit of the embodiment can be used for either type of system.

\<Nonwoven fabric\>

[Nonwoven fabric structure]

The nonwoven fabric to be used in the absorbent pad for an immunochromatographic diagnosis kit of the embodiment is not particularly restricted and may be a spunbonded nonwoven fabric, meltblown nonwoven fabric, wetlaid nonwoven fabric, dry nonwoven fabric, dry pulp nonwoven fabric, flash-spun nonwoven fabric or open-fiber nonwoven fabric. A nonwoven fabric composed of ultrafine fibers is particularly preferred to be a meltblown nonwoven fabric or flash-spun nonwoven fabric.

[Basis weight]

The basis weight of the nonwoven fabric used in the absorbent pad for an immunochromatographic diagnosis kit of the embodiment is 20 g/m$^2$ to 200 g/m$^2$. A basis weight of greater than 200 g/m$^2$ will be unsuitable due to excessive thickness. From the viewpoint of effect on the kit structure and economy there is absolutely no need for a large basis weight. The basis weight is therefore preferably 190 g/m$^2$ or lower and more preferably 180 g/m$^2$. However, a basis weight of less than 20 g/m$^2$ will not be able to ensure that the absorbent pad has sufficient liquid absorption, while in terms of the nonwoven fabric structure it will produce significant inconsistencies in fiber coarseness, leading to poor texture quality as well and consequently greater variation in TL color intensity. The basis weight is therefore preferably 30 g/m$^2$ or greater and more preferably 40 g/m$^2$ or greater.

[Thickness]

The thickness of the nonwoven fabric used in the absorbent pad for an immunochromatographic diagnosis kit of the embodiment is 0.06 mm to 1.20 mm. In regard to the upper limit for the thickness, if it is attempted to produce a nonwoven fabric having a thickness exceeding 1.20 mm, the basis weight and fiber size will deviate from the prescribed ranges, leading to loss of homogeneity in the pore size distribution or texture in either case. The thickness is preferably 0.10 mm or lower and more preferably 0.90 mm or lower. If it is less than 0.06 mm, however, the excessively small thickness will make it difficult to obtain sufficient liquid absorption for an absorbent pad and will not allow strength to be ensured, leading to problems in terms of drafting of the kit. Since this also impairs the texture, the thickness is preferably 0.08 mm or greater and more preferably 0.10 mm or greater.

[Mean fiber diameter]

The mean fiber diameter of the nonwoven fabric used in the absorbent pad for an immunochromatographic diagnosis kit of the embodiment is 0.7 $\mu$m to 5.0 $\mu$m. If the mean fiber diameter exceeds 5.0 $\mu$m the average pore size will increase and the uniformity of the pore size distribution will simultaneously decrease, leading to large variation in TL color intensity. The mean fiber diameter is therefore preferably 4.0 $\mu$m or smaller and more preferably 3.0 $\mu$m or smaller. Moreover, although a finer and more uniform mean fiber diameter results in a denser and more uniform pore size structure, a fiber size that is too small will result in a low basis weight during production and a thinner film thickness, making it difficult to ensure sufficient liquid absorption and strength as an absorbent pad and, in connection with the basis weight, producing notable inconsistencies in fiber coarseness, and greater variation in TL color intensity. The mean fiber diameter is therefore preferably 0.9 $\mu$m or greater and more preferably 1.0 $\mu$m or greater.

[Average pore size]

The average pore size (Dave) of the nonwoven fabric used in the absorbent pad for an immunochromatographic diagnosis kit of the embodiment is 2.0 $\mu$m to 15.0 $\mu$m. If the average pore size is greater than 15.0 $\mu$m the liquid absorption speed will be too rapid for an absorbent pad, considering absorption of liquids by capillary movement, such that liquids may not be properly absorbed depending on the composition of the developing liquid, which will result in variation in absorption and thus increased variation in TL color intensity. The average pore size is therefore preferably 13.5 $\mu$m or smaller and more preferably 12.0 $\mu$m or smaller. If the average pore size is less than 2.0 $\mu$m the liquid absorption speed will decrease causing the TL development time to be retarded by that amount, which is a disadvantage because the time for assessment will be lengthened. The average pore size is therefore preferably 2.5 $\mu$m or greater and more preferably 3.5 $\mu$m or greater.

[Pore size distribution]

The pore size distribution of a nonwoven fabric to be used for an absorbent pad for an immunochromatographic diagnosis kit of the embodiment preferably satisfies the following formulas:

$$\mathrm{Dmax/Dave} < 2.00$$

$$\mathrm{Dmax/Dmin} < 3.50$$

{where Dmax is the maximum pore size ($\mu$m), Dave is the average pore size ($\mu$m), and Dmin is the minimum pore size ($\mu$m)}.

Dmax/Dave < 2.00 is preferred, and Dmax/Dave < 1.50 is more preferred. Dmax/Dave = 1 is the theoretical ideal state of a pore size distribution in which the sizes of pores formed by the fibers of the nonwoven fabric are completely equal. When Dmax/Dave $\geq$ 2.00, the pore size distribution is highly irregular and variation of the TL color intensity is large, which is unsuitable for use as an absorbent pad.

Dmax/Dmin < 3.00 is preferred, and Dmax/Dmin < 2.50 is more preferred. Dmax/Dmin = 1 is the theoretical ideal state of a pore size distribution in which the sizes of pores formed by the fibers of the nonwoven fabric are completely equal. When Dmax/Dmin $\geq$ 3.50, the pore size distribution is highly irregular and variation of the TL color intensity is large, which is unsuitable as it raises the risk of false diagnosis.

[Formation index]

The formation index of the nonwoven fabric used in the absorbent pad for an immunochromatographic diagnosis kit of the embodiment is preferably 90 or lower. A smaller formation index indicates a more uniform nonwoven fabric structure, while a larger formation index indicates a more non-uniform nonwoven fabric structure. The formation index can be adjusted by the basis weight and thickness during production of the nonwoven fabric, as well as by press working after the woven fabric has been produced. In terms of the effect of the formation index on absorbent pad performance, a larger formation index increases variation in the nonwoven fabric structure, resulting in greater variation in TL color intensity and increased risk of false diagnosis. When fabricating a narrow immunochromatographic diagnosis kit (such as a kit with a 2.0 to 3.0 mm width), the nonwoven fabric must be cut more thinly, and consequently any non-uniformity of the nonwoven fabric structure, i.e. difference between coarse portions and dense portions, will affect performance to a greater degree. The formation index is therefore more preferably 75 or lower and even more preferably 60 or lower.

[Thermoplastic resin]

The thermoplastic resin forming the fibers of the nonwoven fabric used in the absorbent pad for an immunochromatographic diagnosis kit of the embodiment is hydrophobic, and may be a polyolefin-based resin, polyester-based resin or polyamide-based resin. Specific examples include $\alpha$-olefins such as ethylene, propylene, 1-butene, 1-hexene, 4-methyl-1-pentene or 1-octene, either alone or as their polyolefin copolymers such as high-pressure method low-density polyethylene, linear low-density polyethylene (LLDPE), high-density polyethylene, polypropylene (propylene homopolymer), polypropylene random copolymer, poly-1-butene, poly-4-methyl-1-pentene, ethylene/propylene random copolymer, ethylene-1-butene random copolymer, or propylene-1-butene random copolymer, polyesters (such as polyethylene terephthalate, polybutylene terephthalate and polyethylene naphthalate), polyamides (such as nylon-6, nylon-66 and poly-meta-xylene adipamide), polyvinyl chloride, polyimide, ethylenevinyl acetate copolymer, polycarbonate, polystyrene, ionomers, and mixtures of the same. Polyethylene terephthalate, polybutylene terephthalate, polypropylene and polyethylene are preferred from the viewpoint of heat and moisture stability and general use. Nonwoven fabrics made of these resins may also be used in combination.

[Hydrophilicizing treatment]

A nonwoven fabric used in the absorbent pad for an immunochromatographic diagnosis kit of the embodiment is subjected to hydrophilicizing treatment and must have a degree of hydrophilization within the range specified below. Most common thermoplastic resins are hydrophobic and absorb virtually no water even when formed into nonwoven fabric structures. For this embodiment, a nonwoven fabric made of hydrophobic thermoplastic resin fibers is used and subjected to hydrophilicizing treatment, whereby it exhibits performance that allows variation in water absorption to be reduced to an absolute minimum. The method of hydrophilization is not particularly restricted and may be a publicly known method, with examples of physical processing methods including hydrophilization by corona treatment and plasma treatment, and examples of chemical processing methods including introduction of surface functional groups, among which are methods of oxidizing treatment to introduce sulfonic acid or carboxylic acid groups, or dip-nip or spray coating methods for impregnation or coating of the nonwoven fabric with a treatment agent, which may be a water-soluble polymer such as polyvinyl alcohol (PVA), a polyester-based resin, polystyrenesulfonic acid or polyglutamic acid and/or a surfactant such as a nonionic surfactant, anionic surfactant, cationic surfactant or zwitterionic surfactant. Suitable hydrophilization processing methods and conditions, such as the use of treatment agents and introduction of functional groups, may be selected in consideration of the water absorbing property required for the absorbent pad.

[Degree of hydrophilization]

The degree of hydrophilization is an index of the water absorption capacity of the absorbent pad for an immunochromatographic diagnosis kit, and it can be evaluated by the Byreck method, dropping method or precipitation method which are commonly used for moisture absorption testing, or by an immunochromatographic liquid absorption test. The degree of hydrophilization of a nonwoven fabric used in an absorbent pad for an immunochromatographic diagnosis kit of the embodiment is a water absorption height of 50 mm to 180 mm based on the Byreck method according to JIS-L1907. If the degree of hydrophilization is less than 50 mm it will not be possible to ensure a sufficient water absorbing property as an absorbent pad for developing and retaining developing solutions, for example. The degree of hydrophilization is therefore preferably 60 mm or greater and more preferably 70 mm or greater. The upper limit for the degree of hydrophilization is not particularly limited but may be 180 mm or lower. A water absorption height of greater than 180 mm may lead to an excessive moisture absorption speed and lower sensitivity, potentially causing development and particle flow to become non-uniform. The degree of hydrophilization is therefore preferably 165 mm or lower and more preferably 150 mm or lower.

[Layering]

A nonwoven fabric used in the absorbent pad for an immunochromatographic diagnosis kit of the embodiment may also be in layered form, depending on the particular use. When a nonwoven fabric is to be layered, any of various methods may be used without any particular limitations, including a heat sealing method such as heat embossing or ultrasonic fusion, a mechanical tangling method such as needle punching or water jet, a bonding method with a hotmelt adhesive or urethane-based adhesive agent, or extrusion lamination.

<Immunochromatography>

The term "diagnostic method", in the context of using an immunochromatographic diagnosis kit with an absorbent pad for an immunochromatographic diagnosis kit according to the embodiment, refers to any of various types of diagnosis using an immunochromatographic diagnosis kit. The target of diagnosis is not particularly restricted, and the kit may be used for examination of various diagnostic targets including examination of humans, animals, food or plants, or environmental monitoring. In a common diagnosis procedure, a specimen sample is harvested from the target of examination, subjected to pretreatment such as extraction and filtration if necessary, and dropped onto the sample pad, a prescribed time is allowed to elapse from the start of the examination, and the diagnosis results are judged based on the color difference produced by the presence or absence of the substance to be examined. There is naturally no limitation to this procedure, and application may be made to diagnoses by similar procedures and principles. Preferably, the specimen sample is filtered beforehand to remove the excess foreign matter or contaminants, in order to allow more rapid diagnosis and to improve the diagnosis precision.

[Immunochromatographic diagnosis kit]

The immunochromatographic diagnosis kit of the embodiment is one that detects the presence or absence of a substance to be examined in various specimens. The type of diagnosis kit may be a lateral flow system or a flow-through system. It is not particularly restricted so long as it uses a labeling reagent and absorbent pad, but it is preferably a lateral flow system. Lateral flow systems include dip stick types and cassette types, but there is no particular restriction on the type. The construction of the diagnosis kit is not particularly restricted, and may be any construction commonly employed in the field. The types of members are not particularly restricted so long as they are ones that are employed in the field, and examples include the (a) absorbent pad, (b) nitrocellulose membrane, (c) conjugate pad (antibody-sensitized labeling reagents), (d) sample pad and (e) mount board shown in Fig. 1. Some of these members may be omitted if necessary.

**[(a) Absorbent pad]**

As shown in Fig. 1, the (a) absorbent pad is the portion that finally absorbs the specimen that is to be measured by immunochromatography. As mentioned above, cellulose filter paper, paper, glass fibers and long glass fibers are used as common absorbent pads in the prior art.

[Chromatographic medium]

The chromatographic medium to be used in the immunochromatographic diagnosis kit is not particularly restricted, and various commonly used chromatographic media may be used. A specific example is (b) a nitrocellulose membrane.

**[(c) Conjugate pad]**

A conjugate pad is a portion that dries and immobilizes labeled particles of an antibody-sensitized labeling reagent, for example. Common conjugate pads include nonwoven fabrics and woven fabrics composed of glass fibers, long glass fibers, acrylic fibers or PET fibers alone or in combinations. It may also be subjected to pretreatment if necessary.

**[(d) Sample pad]**

The "sample pad" is the portion that first receives a specimen that is to be measured by immunochromatography. Common sample pads include those made of cellulose filter paper, paper, glass fibers, long glass fibers, acrylic fibers and nylon fibers, and various woven fabrics. It may also be subjected to pretreatment if necessary. For example, it may be subjected to treatment for pre-addition of a buffering solution, a surfactant, a protein, a reagent to trap contaminants in the specimen sample, an antiseptic agent, an antimicrobial agent, an antioxidant, a humectant or the like.

[Labeling reagent]

A labeling reagent to be fixed on the conjugate pad is a particulate substance that is insoluble in water or a buffering solution and that supports a pigment or dye. The material composing the particles is not particularly restricted, and examples of such labeling reagents include metal colloid particles such as gold colloid, platinum colloid, silver colloid, selenium colloid and the like, colored latex particles including colored styrene-based latexes such as polystyrene latex, or acrylic acid-based latexes, colored silica particles which are colored silica with a 3-dimensional structure made of silicon atoms and oxygen atoms, colored cellulose particles which are colored cellulose, and labeling reagents or magnetic particles that are direct particles of a colorant such as carbon black. The labeling reagent may also be fluorescent particles.

[Supported substance]

The labeling reagent must support a substance that specifically binds to the substance to be detected, such as an antibody, but there is no particular restriction on the method in which it is supported. For example, it may be supported by physical adsorption, by covalent bonding, or by a combination thereof. The type and amount of substance to be supported is not particularly restricted. The type of substance to be supported is most generally preferred to be an antibody. The supporting method is preferably supporting by physical adsorption from the viewpoint of ease, or supporting by covalent bonding from the viewpoint of stability and performance.

[Object of diagnosis]

There are no particular restrictions on the object of diagnosis with the immunochromatographic diagnosis kit of the embodiment, and specific examples include the following: cancer markers, hormones, infectious diseases, autoimmunity, plasma proteins, TDM, coagulation/fibrinolysis, amino acids, peptides, proteins, genes and cells. More specifically, these include CEA, AFP, ferritin, $\beta$2-microglobulin, PSA, CA19-9, CA125, BFP, elastase-1, pepsinogen-1 and -2, fecal occult blood, urinary $\beta$2-microglobulin, PIVKA-2, urinary BTA, insulin, E3, HCG, HPL, LH, HCV antigen, HBs antigen, HBs antibody, HBc antibody, HBe antigen, HBe antibody, HTLV-1 antibody, HIV antibody, *Toxoplasma* antibody, syphilis, ASO, Influenza A antigen, Influenza A antibody, Influenza B antigen, Influenza B antibody, rotavirus antigen, adenovirus antigen, rotavirus/adenovirus antigen, Group A *Streptococcus,* Gr B *Streptococcus, Candida* antigen, CD bacteria, *Cryptococcus* antigen, cholera bacteria, meningococcus antigen, granulocyte elastase, *Helicobacter pylori* antibody, 0157 antibody, 0157 antigen, *Leptospira* antibody, *Aspergillus* antigen, MRSA, RF, total IgE, LE test, CRP, IgG, A, M, IgD, transferrin, urinary albumin, urinary transferrin, myoglobin, C3·C4, SAA, LP(a), $\alpha$1-AC, $\alpha$1-M, haptoglobin, microtransferrin, APR score, FDP, D dimer, plasminogen, AT3, $\alpha$2PI, PIC, PAI-1, protein C, clotting factor X3, type IV collagen, hyaluronic acid and GHbA1c, as well as various other types of antigens, antibodies, viruses, bacteria, amino acids, peptides, proteins, DNA, cells, allergens, residual pesticides and toxins.

[Method of preparing immunochromatographic diagnosis kit]

A dispersion of the labeling reagent adjusted to a predetermined concentration is prepared, buffer and antibody are added, and the mixture is stirred for a fixed time while controlling the temperature to adsorb the antibody onto the labeling reagent. After stirring for a predetermined time, a blocking agent is further added and stirring is continued for a predetermined time while adjusting the temperature, for blocking of the colored cellulose particles. The blocking agent used may be any of various blocking agents depending on the substance to be examined or the specimen, or the composition of their diluted solution. For washing of the colored labeling reagent after antibody adsorption and blocking, centrifugal separation is carried out, the supernatant liquid containing excess antibody and blocking agent, and the precipitated particles, are separated and the supernatant liquid is removed by decantation. Liquids such as the buffer are added to the precipitated particles, and if necessary dispersion treatment is carried out using ultrasonic waves or the like. The washing by the series of procedures including precipitation by centrifugal separation, removal of the supernatant and addition of liquids, is carried out the necessary number of times, and a dispersion is prepared containing a prescribed concentration of the particles that have been subjected to antibody adsorption and blocking. As necessary, proteins, surfactants or sugars such as sucrose or trehalose are added to the dispersion, and the obtained solution is coated in a prescribed amount onto a long glass fiber conjugate pad and dried to prepare a detection reagent-containing section. Also, if necessary, the regenerated cellulose continuous filament nonwoven fabric may be coated with a buffering solution, a surfactant, a protein, a reagent that traps contaminants in the specimen sample, an antiseptic agent, an antimicrobial agent, an antioxidant, a humectant or the like, and dried to prepare the sample pad. There are also prepared a nitrocellulose membrane-composed chromatographic medium immobilizing an antibody, at a predetermined location, and a cellulose filter paper absorbent pad for absorption of the specimen. These are immobilized on a sheet having an attachment site, known as a "backing sheet", and cut to a predetermined size to prepare the immunochromatographic diagnosis kit.

EXAMPLES

The present invention will now be explained in more specific detail through the following examples and comparative examples, with the understanding that the invention is in no way limited to the examples. All of the procedures that are not described in particular were conducted in an environment with a temperature of 23°C and a relative humidity of 55%.
The methods for measuring the physical properties used in the Examples will be explained first.

<Thickness (mm)>

The thickness of the nonwoven fabric was measured by the thickness test of JIS-L1096, with a load of 1.96 kPa (units: mm).

<Basis weight (g/m$^2$)>

A nonwoven fabric with an area of at least 0.5 m$^2$ was dried at 105°C to a fixed weight, and then allowed to stand for 16 hours or longer in a thermostatic chamber at 20°C, 65% RH, the weight was measured, and the weight per unit area of the nonwoven fabric was measured and recorded as the basis weight of the nonwoven fabric.

<Mean fiber diameter ($\mu$m)>

Apparatus model: A JSM-6510 by JEOL Corp. was used. The obtained nonwoven fabric was cut to 10 cm $\times$ 10 cm and vertically pressed on a steel plate at 60°C, at a pressure of 0.30 MPa for 90 seconds, after which the nonwoven fabric was vapor deposited with platinum. An SEM was used for photographing under conditions with an acceleration voltage of 15 kV and a working distance of 21 mm. The photograph magnification was 10,000x for filaments with mean fiber diameters of less than 0.5 $\mu$m, 6000x for filaments with mean fiber diameters of 0.5 $\mu$m or greater and less than 1.5 $\mu$m, and 4000x for filaments of 1.5 $\mu$m or greater. The visual field at each photograph magnification was 12.7 $\mu$m $\times$ 9.3 $\mu$m for 10,000x, 21.1 $\mu$m $\times$ 15.9 $\mu$m for 6000x and 31.7 $\mu$m $\times$ 23.9 $\mu$m for 4000x. A photograph was taken of 100 random fibers, and all of their fiber diameters were photographed. Fibers that were fused in the fiber length direction were not measured. The weight average fiber diameter (Dw) determined by the following formula:

$$Dw = \sum Wi \cdot Di = \sum(Ni \cdot Di^2)/(Ni \cdot Di)$$

{where Wi = weight percentage of fiber diameter Di = Ni·Di/∑Ni·Di} was recorded as the mean fiber diameter (μm).

<Measurement of Dmax, Dave and Dmin>

Apparatus model: An Automated Perm Porometer (Porous Material Automatic Pore Size Distribution Measurement System) by Porous Materials, Inc. was used.

The nonwoven fabric sample is cut to φ25 mm with a punching blade and dipped in GALWICK reagent solution for 1 hour of deaeration. The sample is then set and air pressure is applied. Since the GALWICK reagent solution overcomes liquid surface tension within the capillary tube and is extruded, measuring the pressure at that time allows the maximum pore size Dmax (μm), the average pore size Dave (μm) and the minimum pore size Dmin (μm) to be determined from the Washburn formula, derived from the type of capillary tube.

<Formation index>

Apparatus model: An FMT-MIII by Nomura Shoji Co., Ltd. was used.

Before setting the sample, the amount of transmitted light during light source ON and OFF periods was measured with a CCD camera. A nonwoven fabric cut to A4 size was then set, and the amount of transmitted light was measured in the same manner, and the mean transmittance, mean absorbance and standard deviation (absorbance variation) were determined. The formation index can be determined as standard deviation ÷ mean absorbance × 10. The formation index correlates very well with visual inspection, and it is the most simple representation of formation of a nonwoven fabric. A lower formation index corresponds to better formation, and a larger value with poorer formation.

<Degree of hydrophilization>

The water absorption height was measured by the Byreck method, according to JIS-L1907. Specifically, five test pieces each with a size of about 200 mm × 25 mm were prepared. After using a pin to anchor the test piece onto a horizontal bar supported on the water surface of a water-containing water tank, the horizontal bar was lowered so that 20 mm ±2 mm of the lower end of the test piece was soaked with the water, and it was allowed to stand in that position for 10 minutes. The height of the water that rose by capillary movement was then measured with a scale to the nearest 1 mm. The average value for the water absorption heights of each of the five test pieces was recorded as the degree of hydrophilization.

<Immunochromatographic diagnosis kit background (BG coloration)>

The immunochromatographic diagnosis kit cut to a suitable width was placed in a plastic housing. A 66 mM PBS solution at pH 7.4 containing 1 wt% BSA was also prepared as a negative specimen. Using the obtained housing-containing diagnosis kit, 100 μL of negative specimen was added dropwise into sample dropping unit of the diagnosis kit, and after 5 minutes, a C 10066-10 Immunochromato-Reader by Hamamatsu Photonics, K.K. was used to measure the color intensity at one point between TL and CL (units: mABS). A color intensity of 20 mABS or greater was judged to be BG coloration. The reason for the threshold of 20 mABS is because the coloration can be reliably confirmed by sight if the value is 20 mABS or greater.

<Coloration time for test line (TL) with immunochromatographic diagnosis kit>

For measurement of the TL coloration time, human chorionic gonadotropin (hereunder, "hCG") was used as the substance to be examined, diluting the hCG with 66 mM phosphate buffered saline (hereunder, "PBS") at pH 7.4 containing 1 wt% bovine serum albumin (hereunder, "BSA"), to prepare a positive specimen with an hCG concentration of 10 mIU/ml. A 100 μl portion of the positive specimen was dropped into the sample dropping unit of the diagnosis kit, and measurement was performed with a similar Immunochromato-Reader every 20 seconds thereafter, determining the change in the TL over time. The reason for measurement every 20 seconds is that just less than 20 seconds is necessary for each measurement. The time at which the color intensity of the TL obtained by the Immunochromato-Reader was 20 mABS or greater was recorded. The reason for the index of 20 mABS is because the presence of a TL becomes visible at 20 mABS or greater. The measurement was conducted a total of 30 times, and the average time (units: seconds) was recorded as the TL coloration time.

<Test line (TL) color intensity>

For measurement of the TL color intensity, 100 $\mu$l of positive specimen was dropped into the sample dropping unit of the diagnosis kit in the same manner as described above, and the test line color intensity (mABS) after 5 minutes was measured with an Immunochromato-Reader. The measurement was conducted a total of 30 times, and the average color intensity (mABS) was recorded as the TL color intensity.

The standard deviation of the TL color intensity was also determined, and the index %CV representing the reproducibility was calculated by the following formula:

$$\%CV = (TL\ color\ intensity\ standard\ deviation/TL\ color\ intensity) \times 100.$$

[Example 1]

<Fabrication of thermoplastic nonwoven fabric>

A polyethylene terephthalate resin was used as the main resin and melted with an extruder, and then extruded through a spinneret nozzle with a nozzle diameter of 0.3 mm at a single-hole throughput of 0.12 g/min. During extrusion of the thermoplastic resin, spinning gas was used with the conditions were set to a spinning gas temperature of 370°C and a spinning gas pressure of 0.075 MPa, to fabricate a polyethylene terephthalate nonwoven fabric. The obtained nonwoven fabric had a basis weight of 80 g/m$^2$, a thickness of 0.22 mm and a mean fiber diameter of 1.9 $\mu$m. When the pore size and formation index were measured by the methods described above, the values of Dmax: 5.64 $\mu$m, Dave: 4.10 $\mu$m, Dmin: 3.23 $\mu$m and formation index: 49 were obtained.

<Hydrophilic treatment>

The fabricated nonwoven fabric was hydrophilized using a dip nip method. Specifically, a mixed hydrophilic treatment agent comprising an anionic surfactant and water was placed in a treatment bath, a supply sheet of the fabricated thermoplastic nonwoven fabric was dipped and mangled for contraction to a predetermined percentage of contraction, after which it was dried and heat treated with a tenter dryer. After hydrophilic treatment, the degree of hydrophilization was measured by the Byreck method to be 109 mm.

<Preparation of antibody-sensitized gold colloid particles>

To 2500 $\mu$l of a gold colloid particle suspension (product of Tanaka Holdings Co., Ltd., average particle diameter:40 nm, particle concentration: 0.006 wt%, average particle diameter: 40 nm) there was added 600 $\mu$l of phosphate buffer (50 mM, pH 7.0), and then 200 $\mu$l of a 0.1% aqueous solution of anti-hCG-$\alpha$ mouse antibody (product of Fitzgerald Co., monoclonal antibody) was added and the mixture was stirred with a vortex. It was then further stirred for 10 minutes while controlling the temperature to 25°C. To the suspension there were added 300 $\mu$l of a 1% aqueous PEG solution and 600 $\mu$l of a 10% aqueous BSA solution (pH 9.0, containing 50 mM boric acid), and the mixture was further stirred with a vortex. Centrifugal separation was then carried out (10,000 g, 30 minutes) and the supernatant liquid was removed. To the residue there was added 11,000 $\mu$l of a 1% aqueous BSA solution (containing 0.05% PEG, 150 mM NaCl, pH 8.2, 20 mM Tris), and the mixture was stirred with a vortex. Centrifugal separation was then carried out (10,000 g, 30 minutes) and the supernatant liquid was removed. To the residue there was added 900 $\mu$l of a 1% aqueous BSA solution (containing 0.05% PEG, 150 mM NaCl, pH 8.2, 20 mM Tris), and ultrasonic treatment was carried out for 30 seconds.

<Impregnation of conjugate pad with labeling reagent, and drying>

A long glass fiber conjugate pad (#8951 by Ahlstrom Co.) was cut to a height of 10 mm and a length of 300 mm. It was then coated evenly with 1020 $\mu$l of an antibody-sensitized gold colloid particle dispersion, and dried at 50°C for 60 minutes.

<Pretreatment of sample pad>

A sample pad (C048 by Millipore) was impregnated with an excess of PBS buffer (66 mM, pH 7.4) containing 2.0 wt% BSA (A7906 by Sigma-Aldrich Japan, KK.) and 2.0 wt% Tween-20$^R$, and after removing the excess solution, it was dried at 50°C for 60 minutes. It was then cut into a shape with a height of 18 mm and a length of 300 mm.

<Preparation of capture antibody-coated nitrocellulose membrane>

A nitrocellulose membrane (SHF0900425 by Millipore) was cut into a shape with a height of 25 mm and a length of 300 mm. A liquid coating applicator (300DS by Musashi Engineering, Inc.) was used for coating of a PBS solution (66 mM, pH 7.4) containing 0.1 wt% anti-hCG-$\beta$ mouse antibody (6601 by MedixBiochemica) at a proportion of 0.1 $\mu$l/mm onto a 7 mm-high section. Next, a PBS solution (66 mM, pH 7.4) containing 0.1 wt% anti-mouse-rabbit antibody (Z0259 by Daco Co.) was coated at a proportion of 0.1 $\mu$l/mm onto a 12 mm-high section. It was then dried at 37°C for 30 minutes.

<Preparation of immunochromatographic diagnosis kit>

The capture antibody-coated nitrocellulose membrane, absorbent pad, labeling reagent-containing conjugate pad and sample pad were attached onto a backing card (AR9020 by Adhesives Research Co.). The layout was then cut to a 5 mm width with a cutter, to obtain an immunochromatographic diagnosis kit with a width of 5 mm and a height of 60 mm.

<Performance evaluation of immunochromatographic diagnosis kit>

The performance of the obtained immunochromatographic diagnosis kit was evaluated. The results are shown in Table 1 below.

For performance evaluation of absorbent pads with a thickness of 0.2 mm or greater using an immunochromatographic diagnosis kit width of 5 mm in the Examples and Comparative Examples, since the amount of developing solution that can be developed is about 80 to 120 $\mu$l, evaluation was actually conducted with development of 100 $\mu$l, as shown in Table 1. When using a kit width of 2.5 mm or an absorbent pad thickness of less than 0.2 mm, since the amount of developing solution that can be developed is about 30 to 80 $\mu$l, evaluation was actually conducted with development of 50 $\mu$l, as also shown in Table 1.

[Example 2]

An immunochromatographic diagnosis kit was prepared in the same manner as Example 1, except that production was to a thermoplastic nonwoven fabric basis weight of 90 g/m$^2$ and a mean fiber diameter of 2.1 $\mu$m, and the performance was evaluated. The results are shown in Table 1 below.

[Example 3]

An immunochromatographic diagnosis kit was prepared in the same manner as Example 1, except that production was to a thermoplastic nonwoven fabric mean fiber diameter of 4.7 $\mu$m, and the performance was evaluated. The results are shown in Table 1 below.

[Example 4]

An immunochromatographic diagnosis kit was prepared in the same manner as Example 1, except that production was to a thermoplastic nonwoven fabric basis weight of 120 g/m$^2$, and the performance was evaluated. The results are shown in Table 1 below.

[Example 5]

An immunochromatographic diagnosis kit was prepared in the same manner as Example 1, except that production was to a thermoplastic nonwoven fabric basis weight of 185 g/m$^2$, and the performance was evaluated. The results are shown in Table 1 below.

[Example 6]

An immunochromatographic diagnosis kit was prepared in the same manner as Example 1, except that production was with adjustment of the hydrophilic agent concentration during hydrophilicizing treatment of the thermoplastic nonwoven fabric to a degree of hydrophilization of 171 mm, and the performance was evaluated. The results are shown in Table 1 below.

[Example 7]

An immunochromatographic diagnosis kit was prepared in the same manner as Example 1, except that production was with the degree of hydrophilization of the thermoplastic nonwoven fabric at 72 mm, similar to Example 6, and the performance was evaluated. The results are shown in Table 1 below.

[Example 8]

An immunochromatographic diagnosis kit was prepared in the same manner as Example 1, except that production was with the basis weight and thickness of the thermoplastic nonwoven fabric adjusted, and with a formation index of 95, and the performance was evaluated. The results are shown in Table 1 below.

[Example 9]

An immunochromatographic diagnosis kit was prepared in the same manner as Example 1, except that the thermoplastic nonwoven fabric was produced using polypropylene, and the performance was evaluated. The results are shown in Table 1 below.

[Example 10]

An immunochromatographic diagnosis kit was prepared in the same manner as Example 1, except for using the hydrophilized thermoplastic nonwoven fabric used in Example 2 in layered form, and the performance was evaluated. The results are shown in Table 1 below.

[Example 11]

An immunochromatographic diagnosis kit was prepared in the same manner as Example 1, except that production was to a thermoplastic nonwoven fabric basis weight of 45 g/m$^2$, a thickness of 0.14 mm and a mean fiber diameter of 1.6 $\mu$m, and the performance was evaluated with 50 $\mu$l as the drip volume for each specimen. The results are shown in Table 1 below.

[Example 12]

An immunochromatographic diagnosis kit was prepared in the same manner as Example 1, except that production was to a thermoplastic nonwoven fabric basis weight of 40 g/m$^2$, a thickness of 0.10 mm and a mean fiber diameter of 1.0 $\mu$m, and the performance was evaluated in the same manner as Example 11. The results are shown in Table 1 below.

[Example 13]

An immunochromatographic diagnosis kit was prepared in the same manner as Example 1, except that production was to a thermoplastic nonwoven fabric basis weight of 20 g/m$^2$, a thickness of 0.06 mm and a mean fiber diameter of 0.9 $\mu$m, and the performance was evaluated in the same manner as Example 11. The results are shown in Table 1 below.

[Example 14]

An immunochromatographic diagnosis kit was prepared in the same manner as Example 2, and the performance of a piece cut to a 2.5 mm width was evaluated in the same manner as Example 11. The results are shown in Table 1 below.

[Explanation of Examples 1 to 14]

For all of Examples 1 to 14, the absorbent pads used hydrophilized thermoplastic resins having properties within the ranges specified by claim 1, whereby it was possible to obtain immunochromatographic diagnosis kits reliably having no BG coloration after 5 minutes (<20 mABS), low variation in TL color intensity and low variation between products, as shown in Table 1 below.

[Comparative Example 1]

An immunochromatographic diagnosis kit was prepared in the same manner as Example 1, except that production was to a thermoplastic nonwoven fabric basis weight of 220 g/m$^2$ but with a thickness of 0.55 mm, and the performance was evaluated. The results are shown in Table 1 below. As clearly seen by the results in Table 1, the nonwoven fabric had considerable pore clogging and insufficient water absorption, and therefore produced BG coloration after 5 minutes and had an increased %CV for TL color intensity.

[Comparative Example 2]

An immunochromatographic diagnosis kit was prepared in the same manner as Example 1, except that production was to a thermoplastic nonwoven fabric basis weight of 80 g/m$^2$ but with a thickness of 1.30 mm, and the performance was evaluated. The results are shown in Table 1 below. As clearly seen by the results in Table 1, the structure was very coarse due to excessive thickness compared to its basis weight, and therefore the formation index and the %CV for TL color intensity were significantly increased.

[Comparative Example 3]

An immunochromatographic diagnosis kit was prepared in the same manner as Example 1, except that production was to a thermoplastic nonwoven fabric mean fiber diameter of 5.5 μm, and the performance was evaluated. The results are shown in Table 1 below. As clearly seen by the results in Table 1, the fiber diameter was too thick and the formation index was poor, with significantly increased %CV for TL color intensity, similar to Comparative Example 2.

[Comparative Example 4]

An immunochromatographic diagnosis kit was prepared in the same manner as Example 1, except that production was to a thermoplastic nonwoven fabric degree of hydrophilization of 48 mm, and the performance was evaluated. As clearly seen by the results in Table 1, BG coloration was present after 5 minutes similar to Comparative Example 1, and the TL coloration time was also significantly slower. The %CV for TL color intensity was also significantly increased.

[Comparative Example 5]

An immunochromatographic diagnosis kit was prepared in the same manner as Example 1 except for using a thick cellulose absorbent pad, and the performance was evaluated. The results are shown in Table 1 below. As clearly seen by the results in Table 1, BG coloration was present after 5 minutes, and the %CV for TL color intensity was also significantly increased.

[Comparative Example 6]

An immunochromatographic diagnosis kit was prepared in the same manner as Example 1 except for using a thin cellulose absorbent pad, and the performance was evaluated. The results are shown in Table 1 below. As clearly seen by the results in Table 1, BG coloration was present after 5 minutes, and the %CV for TL color intensity was also significantly increased.

[Comparative Example 7]

An immunochromatographic diagnosis kit was prepared in the same manner as Example 1 except for using a long glass fiber absorbent pad, and the performance was evaluated. The results are shown in Table 1 below. As clearly seen by the results in Table 1, BG coloration was present after 5 minutes, similar to Comparative Example 1, and the %CV for TL color intensity was also significantly increased. In addition, dust was visibly generated during fabrication of the immunochromatographic diagnosis kit, especially during the cutting step.

[Comparative Example 8]

An immunochromatographic diagnosis kit was prepared in the same manner as Example 1, except that production was to a thermoplastic nonwoven fabric basis weight of 15 g/m$^2$ and a thickness of 0.05 mm, and the performance was evaluated in the same manner as Example 11. The results are shown in Table 1 below. As clearly seen by the results in Table 1, liquid absorption of the absorbent pad for the developing solution was inadequate, preventing development.

[Comparative Example 9]

An immunochromatographic diagnosis kit was prepared in the same manner as Example 1, except that production was to a thermoplastic nonwoven fabric mean fiber diameter of 0.6 μm, and the performance was evaluated in the same manner as Example 11. The results are shown in Table 1 below. As clearly seen by the results in Table 1, the liquid absorption was inadequate preventing development, similar to Comparative Example 8.

[Comparative Example 10]

An immunochromatographic diagnosis kit was prepared using a thick cellulose absorbent pad and with a kit width of 2.5 mm, and the performance was evaluated in the same manner as Example 11, but the thickness was too large making it impossible to be cut into a narrow section of 2.5 mm, and therefore a kit could not be fabricated and performance evaluation was not possible.

[Comparative Example 11]

An immunochromatographic diagnosis kit was prepared using a thin cellulose absorbent pad and with a kit width of 2.5 mm, and the performance was evaluated in the same manner as Example 11. The results are shown in Table 1 below. As clearly seen by the results in Table 1, BG coloration was present after 5 minutes, and the %CV for TL color intensity was significantly increased.

[Comparative Example 12]

An immunochromatographic diagnosis kit was prepared using a long glass fiber absorbent pad and with a kit width of 2.5 mm, and the performance was evaluated in the same manner as Example 11. The results are shown in Table 1 below. As clearly seen by the results in Table 1, BG coloration was present after 5 minutes, similar to Comparative Example 1, and the %CV for TL color intensity was significantly increased, being even poorer than the %CV with a 5 mm width (Comparative Example 7). In addition, dust was visibly generated during fabrication of the immunochromatographic diagnosis kit, especially during the cutting step.

[Table 1]

| | | Kit construction | | Absorbent pad | | | | | | | | | | | | Immunochromatography evaluation results | | | | | Remark |
|---|---|---|---|---|---|---|---|---|---|---|---|---|---|---|---|---|---|---|---|---|---|
| | | Developable amount of developing solution | Kit width | Material | Layering | Basis weight | Thickness | Mean fiber diameter | Average pore size | Pore size distribution | | Formation index | Degree of hydrophilization | BG coloration (after 5 min, mABS) | TL coloration time (sec) | TL intensity hCG 10mIU/ml | | | | |
| | | μl | Mm | | | G/m² | Mm | μm | μm | Dmax/Dave | Dmax/Dmin | | Mm | | | MABS | Standard deviation (mABS) | %CV | | |
| EP 3 919 908 A1 | 1 | | 5.0 | Polyethylene terephthalate | 1 layer | 80 | 0.22 | 1.9 | 4.1 | 1.37 | 1.74 | 49 | 109 | 2.3 | 48 | 323 | 6.1 | 1.9 | |
| | 2 | | 5.0 | Polyethylene terephthalate | 1 layer | 90 | 0.42 | 2.1 | 6.1 | 1.41 | 1.90 | 54 | 98 | 1.7 | 47 | 325 | 6.4 | 2.0 | |
| | 3 | | 5.0 | Polyethylene terephthalate | 1 layer | 90 | 0.24 | 4.7 | 9.6 | 1.34 | 2.03 | 80 | 115 | 2.5 | 38 | 308 | 10.7 | 3.5 | |
| | 4 | | 5.0 | Polyethylene terephthalate | 1 layer | 120 | 0.81 | 2.5 | 10.1 | 1.39 | 2.28 | 50 | 142 | 1.3 | 42 | 315 | 7.7 | 2.4 | |

(continued)

| | | Kit construction | | | | Absorbent pad | | | | | | | | | Immunochromatography evaluation results | | | | | Remark |
|---|---|---|---|---|---|---|---|---|---|---|---|---|---|---|---|---|---|---|---|---|
| | | Developable amount of developing solution | Kit width | Material | Layering | Basis weight | Thickness | Mean fiber diameter | Average pore size | Pore size distribution | | Formation index | Degree of hydrophilization | BG coloration (after 5 min, mABS) | TL coloration time (sec) | TL intensity hCG 10mIU/ml | | | |
| | | | | | | | | | | Dmax/Dave | Dmax/Dmin | | | | | MABS | Standard deviation (mABS) | %CV | |
| | | µl | Mm | | | G/m² | Mm | µm | µm | | | | Mm | | | | | | |
| Example | 5 | 80-120 (100) | 5.0 | Polyethylene terephthalate | 1 layer | 185 | 1.06 | 2.0 | 7.1 | 1.34 | 1.98 | 72 | 161 | 1.5 | 49 | 312 | 5.0 | 1.6 | |
| | 6 | | 5.0 | Polyethylene terephthalate | 1 layer | 80 | 0.22 | 1.9 | 4.1 | 1.37 | 1.74 | 49 | 171 | 0.8 | 30 | 264 | 10.6 | 4.0 | |
| | 7 | | 5.0 | Polyethylene terephthalate | 2 layers | 80 | 0.22 | 1.9 | 4.1 | 1.37 | 1.74 | 49 | 72 | 18 | 87 | 270 | 11.7 | 4.3 | |
| | 8 | | 5.0 | Polyethylene terephthalate | 1 layer | 120 | 1.10 | 2.0 | 12.3 | 1.41 | 2.40 | 95 | 139 | 1.7 | 45 | 316 | 17.6 | 5.6 | |
| | 9 | | 5.0 | Polypropylene | 2 layers | 40 | 0.13 | 0.7 | 11.8 | 1.45 | 1.84 | 88 | 153 | 10.1 | 72 | 250 | 11.5 | 4.6 | |

EP 3 919 908 A1

| | | Kit construction | | | | Absorbent pad | | | | | | | | | Immunochromatography evaluation results | | | | | Remark |
|---|---|---|---|---|---|---|---|---|---|---|---|---|---|---|---|---|---|---|---|---|
| | | Developable amount of developing solution | Kit width | Material | Layering | Basis weight | Thickness | Mean fiber diameter | Average pore size | Pore size distribution | | Formation index | Degree of hydrophilization | BG coloration (after 5 min, mABS) | TL coloration time (sec) | TL intensity hCG 10mIU/ml | | | |
| | | | | | | | | | | Dmax/Dave | Dmax/Dmin | | | | | MABS | Standard deviation (mABS) | %CV | |
| | | μl | Mm | | | G/m² | Mm | μm | μm | | | | Mm | | | | | | |
| EP 3 919 908 A1 | 10 | | 5.0 | Polyethylene terephthalate | 2 layers | 180 | 0.81 | 2.1 | 6.3 | 1.32 | 1.98 | 53 | 101 | 0.9 | 50 | 328 | 6.1 | 1.9 | |
| | 11 | | 5.0 | Polyethylene terephthalate | 1 layer | 45 | 0.14 | 1.6 | 6.4 | 1.01 | 1.27 | 56 | 130 | 4.8 | 55 | 320 | 7.0 | 2.2 | |
| | 12 | 30-80 (50) | 5.0 | Polyethylene terephthalate | 1 layer | 40 | 0.10 | 1.0 | 5.9 | 1.36 | 2.14 | 63 | 138 | 9.2 | 58 | 307 | 10.2 | 3.3 | |
| | 13 | | 5.0 | Polyethylene terephthalate | 1 layer | 20 | 0.06 | 0.9 | 4.8 | 1.22 | 1.67 | 72 | 150 | 15 | 68 | 299 | 10.2 | 3.4 | |
| | 14 | | 2.5 | Polyethylene terephthalate | 1 layer | 90 | 0.42 | 2.1 | 6.1 | 1.41 | 1.90 | 54 | 102 | 3.9 | 52 | 318 | 7.6 | 2.4 | |

| | | Kit construction | | Absorbent pad | | | | | | | | | | Immunochromatography evaluation results | | | | | Remark |
|---|---|---|---|---|---|---|---|---|---|---|---|---|---|---|---|---|---|---|---|---|
| | | Developable amount of developing solution | Kit width | Material | Layering | Basis weight | Thickness | Mean fiber diameter | Average pore size | Pore size distribution | | Formation index | Degree of hydrophilization | BG coloration (after 5 min, mABS) | TL coloration time (sec) | TL intensity hCG 10mIU/ml | | | |
| | | | | | | | | | | Dmax/ Dave | Dmax/ Dmin | | | | | MABS | Standard deviation (mABS) | %CV | |
| | | µl | Mm | | | G/m² | Mm | µm | µm | | | | Mm | | | | | | |
| | 1 | | 5.0 | Polyethylene terephthalate | 1 layer | 220 | 0.55 | 4.8 | 3.3 | 1.29 | 1.83 | 68 | 63 | 89 | 141 | 219 | 22.1 | 10.1 | |
| | 2 | | 5.0 | Polyethylene terephthalate | 1 layer | 80 | 1.30 | 1.1 | 16.1 | 1.57 | 4.50 | 132 | 131 | 5.2 | 42 | 305 | 38.8 | 12.7 | |
| | 3 | | 5.0 | Polyethylene terephthalate | 1 layer | 100 | 1.05 | 5.5 | 9.3 | 1.32 | 1.95 | 123 | 125 | 72 | 35 | 301 | 35.8 | 11.9 | |
| | 4 | | 5.0 | Polyethylene terephthalate | 1 layer | 80 | 0.22 | 1.9 | 4.1 | 1.37 | 1.74 | 49 | 48 | 71 | 137 | 240 | 25.8 | 10.8 | |
| | 5 | 80-120 (100) | 5.0 | Cellulose | 1 layer | 285 | 0.83 | 5.5 | Not measurable | Not measurable | Not measurable | 50 | 159 | 34 | 45 | 316 | 45.4 | 14.4 | |

EP 3 919 908 A1

19

| | | Kit construction | | | | Absorbent pad | | | | | | | | | Immunochromatography evaluation results | | | | | | |
|---|---|---|---|---|---|---|---|---|---|---|---|---|---|---|---|---|---|---|---|---|---|
| | | Developable amount of developing solution | Kit width | Material | Layering | Basis weight | Thickness | Mean fiber diameter | Average pore size | Pore size distribution | | Formation index | Degree of hydrophilization | BG coloration (after 5 min, mABS) | TL coloration time (sec) | TL intensity hCG 10mIU/ml | | | Remark |
| | | | | | | | | | | Dmax/Dave | Dmax/Dmin | | | | | MABS | Standard deviation (mABS) | %CV | |
| | | μl | Mm | | | G/m² | Mm | μm | μm | | | | Mm | | | | | | |
| | 6 | | 5.0 | Cellulose | 1 layer | 180 | 0.51 | 5.3 | Not measurable | Not measurable | Not measurable | 64 | 143 | 24 | 49 | 319 | 50.1 | 15.7 | |
| | 7 | | 5.0 | Long glass fibers | 1 layer | 74 | 0.51 | 4.2 | Not measurable | Not measurable | Not measurable | 127 | 52 | 54 | 72 | 275 | 47.9 | 17.4 | Generation of dust during kit production |
| Comp. Example | 8 | | 5.0 | Polyethylene terephthalate | 1 layer | 15 | 0.05 | 1.0 | 5.1 | 1.60 | 4.05 | 130 | 148 | 114 | No TL coloration | | | | Poor development |
| | 9 | | 5.0 | Polyethylene terephthalate | 1 layer | 30 | 0.08 | 0.6 | 5.0 | 1.62 | 4.26 | 131 | 139 | 92 | No TL coloration | | | | Poor development |

(continued)

| No. | Kit construction | | Absorbent pad | | | | | | | | | | Immunochromatography evaluation results | | | | | Remark |
|---|---|---|---|---|---|---|---|---|---|---|---|---|---|---|---|---|---|---|
| | Developable amount of developing solution | Kit width | Material | Layering | Basis weight | Thickness | Mean fiber diameter | Average pore size | Pore size distribution | | Formation index | Degree of hydrophilization | BG coloration (after 5 min, mABS) | TL coloration time (sec) | TL intensity hCG 10mIU/ml | | | |
| | | | | | | | | | Dmax/ Dave | Dmax/ Dmin | | | | | MABS | Standard deviation (mABS) | %CV | |
| | μl | Mm | | | G/m² | Mm | μm | μm | | | | Mm | | | | | | |
| 10 | 30-80 (50) | 2.5 | Cellulose | 1 layer | 285 | 0.83 | 5.5 | Not measurable | Not measurable | Not measurable | 50 | 159 | Immunochromatographic evaluation impossible | | | | | Kit cutting during kit production impossible |
| 11 | | 2.5 | Cellulose | 1 layer | 180 | 0.51 | 5.3 | Not measurable | Not measurable | Not measurable | 64 | 143 | 28 | 59 | 307 | 51.6 | 16.8 | |
| 12 | | 2.5 | Long glass fibers | 1 layer | 74 | 0.51 | 4.2 | Not measurable | Not measurable | Not measurable | 127 | 52 | 55 | 94 | 249 | 51.5 | 20.7 | Generation of dust during kit production |

*Values in parentheses are actual addition amounts.

21

INDUSTRIAL APPLICABILITY

An immunochromatographic diagnosis kit using an absorbent pad for an immunochromatographic diagnosis kit of the invention has low variation in color intensity and excellent reproducibility. According to the present invention, a hydrophobic thermoplastic nonwoven fabric is used and subjected to hydrophilicizing treatment, thus allowing the water absorption power of the invention to be controlled and resulting in improved background, while in the production process for the immunochromatographic diagnosis kit as well, a lower amount of fibers are shed and cutting and shaping are easier, compared to long glass fibers, so that a high degree of freedom can be obtained for design of the sizes of pads or kits including their thicknesses and widths, while stability during the production steps can also be ensured. The absorbent pad for an immunochromatographic diagnosis kit of the invention can therefore be suitably used as an absorbent pad for various types of immunochromatographic diagnosis kits.

REFERENCE SIGNS LIST

(a)   Absorbent pad
(b)   Nitrocellulose membrane
(c)   Conjugate pad (including antibody-sensitized labeling reagent)
(d)   Sample pad
(e)   Mount board

**Claims**

1.   An absorbent pad for an immunochromatographic diagnosis kit comprising a nonwoven fabric composed of thermoplastic resin fibers, wherein the nonwoven fabric has a basis weight of 20 to 200 g/m$^2$, a thickness of 0.06 mm to 1.20 mm, a mean fiber diameter of 0.7 $\mu$m to 5.0 $\mu$m, an average pore size of 2.0 $\mu$m to 15.0 $\mu$m and a degree of hydrophilization of 50 mm to 180 mm.

2.   The absorbent pad for an immunochromatographic diagnosis kit according to claim 1, wherein the formation index of the nonwoven fabric is 90 or lower and the pore size distribution satisfies the following formulas:

$$Dmax/Dave < 2.00$$

$$Dmax/Dmin < 3.50$$

{where Dmax is the maximum pore size ($\mu$m), Dave is the average pore size ($\mu$m), and Dmin is the minimum pore size ($\mu$m)}.

3.   The absorbent pad for an immunochromatographic diagnosis kit according to claim 1 or 2, wherein the thermoplastic resin is any one or a combination of more than one from among polyethylene terephthalate, polybutylene terephthalate, polypropylene and polyethylene.

4.   The absorbent pad for an immunochromatographic diagnosis kit according to any one of claims 1 to 3, wherein the nonwoven fabric is a single-layer nonwoven fabric or a layered nonwoven fabric.

5.   An immunochromatographic diagnosis kit using an absorbent pad for an immunochromatographic diagnosis kit according to any one of claims 1 to 4.

# FIG. 1

(e)  (d)  (c)  (b)  (a)

Liquid development direction

## INTERNATIONAL SEARCH REPORT

| International application No. |
| --- |
| PCT/JP2020/003029 |

### A. CLASSIFICATION OF SUBJECT MATTER
Int. Cl. C01N33/543(2006.01)i, C01N33/544(2006.01)i
FI: G01N33/543 521, G01N33/544 Z

According to International Patent Classification (IPC) or to both national classification and IPC

### B. FIELDS SEARCHED

Minimum documentation searched (classification system followed by classification symbols)
Int. Cl. G01N33/543, G01N33/544

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)
JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| A | JP 2013-53897 A (SEIKO EPSON CORP.) 21 March 2013, paragraphs [0029], [0030], [0032] | 1-5 |
| A | JP 2010-256309 A (ASAHI KASEI FIBERS CORP.) 11 November 2010, paragraphs [0002], [0023], [0027] | 1-5 |
| A | JP 2009-63482 A (TEIJIN FIBERS LTD.) 26 March 2009, paragraph [0007], examples 1, 2, 3 | 1-4 |
| A | US 2002/0055126 A1 (SCHAFFLER, Jurgen) 09 May 2002, paragraphs [0010], [0053] | 1-5 |
| A | JP 2015-49158 A (AION KK) 16 March 2015, paragraphs [0024], [0041] | 1-5 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 16.03.2020 | 31.03.2020 |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2020/003029

C (Continuation).  DOCUMENTS CONSIDERED TO BE RELEVANT

| Category* | Citation of document, with indication, where appropriate, of the relevant passages | Relevant to claim No. |
|---|---|---|
| P, A | JP 2019-15583 A (TOYOBO CO., LTD.) 31 January 2019, paragraph [0014] | 3-5 |

Form PCT/ISA/210 (continuation of second sheet) (January 2015)

INTERNATIONAL SEARCH REPORT
Information on patent family members

| Patent Documents referred to in the Report | Publication Date | Patent Family | Publication Date |
|---|---|---|---|
| JP 2013-53897 A | 21.03.2013 | (Family: none) | |
| JP 2010-256309 A | 11.11.2010 | (Family: none) | |
| JP 2009-63482 A | 26.03.2009 | (Family: none) | |
| US 2002/0055126 A1 | 09.05.2002 | EP 001143248 A2 | |
| JP 2015-49158 A | 16.03.2015 | (Family: none) | |
| JP 2019-15583 A | 31.01.2019 | (Family: none) | |

International application No.

PCT/JP2020/003029

**EP 3 919 908 A1**

## REFERENCES CITED IN THE DESCRIPTION

**Patent documents cited in the description**

- JP 6134615 B

- JP 2012189346 A